# EUROPEAN PATENT APPLICATION

(11) **EP 1 876 188 A1**
(43) Date of publication of application: **09.01.2008**
(21) Application number: 06116567.6
(22) Date of filing: 04.07.2006
(51) Int. Cl.: C08F 122/32, A61K 47/48

(54) **Two step miniemulsion process**

(71) Applicant: NanoDel Technologies GmbH, 39120 Magdeburg (DE); Universität Ulm, 89081 Ulm (DE)
(72) Inventor: Landfester, Katharina, 89075 Ulm (DE); Weiss, Clemens, 89134 Blaustein (DE); Kubasch, Julie, 76228 Karlsruhe (DE)
(74) Representative: Behnisch, Werner

(57) **Abstract**

The present invention is directed to a method of producing nanoparticles and nanoparticles obtainable by that method. The invention further relates to a pharmaceutical composition, comprising said nanoparticles and the use of the nanoparticles for the treatment of diseases and conditions, requiring a pharmaceutical agent to cross one or more physiological barriers.

## Description

The present invention is directed to a method of producing nanoparticles and nanoparticles obtainable by that method. The invention further relates to a pharmaceutical composition, comprising said nanoparticles and the use of the nanoparticles for the treatment of diseases and conditions, requiring a pharmaceutical agent to cross one or more physiological barriers.

### Background of the invention

### I. Biological barriers

While many drugs are able to cross biological barriers like the bbb, others do not pass those barriers efficiently or not at all and are only effective when given directly into the target tissue. Thus, many potentially potent drugs are not useful clinically due to their inability to pass biological barriers like the bbb.

A number of approaches have been described in the prior art to increase drug penetration through the biological barriers.

One approach has been to alter the function of the barrier, for example the bbb itself. For instance, osmotic agents, when given peripherally (such as by intravenous injection), result in the opening of the bbb. Further, some drugs acting on the CNS can change the permeability of the bbb for other substances; cholinomimetic arecolines, for instance, have been reported to induce changes of drug penetration through the bbb [Saija, A., Princi, P., De Pasquale, R., Costa, G., "Arecoline but not haloperidol produces changes in the permeability of the blood-brain barrier in the rat." J. Pharm. Pha. 42:135-138 (1990)].

Another approach resides in the modification of the drug molecules themselves. For instance, macromolecules, such as proteins, do not pass the bbb at all. For example, one can first isolate the macromolecule active site, i.e., the portion of the molecule which triggers the biologically desirable event, and then use only this active site. Since size is one of the factors in allowing permeability of the bbb, the reduced size is used in the hope that the smaller molecule can now pass the bbb. Other modifications of macromolecules to attempt passage of the bbb include glycating the proteins, thereby enhancing their permeability of the bbb, or forming a prodrug.

U.S. Patent No. 5,260,308 discusses glycating proteins, while U.S. Patent No. 4,933,324 and WO 89/07938 disclose the formation of a prodrug. These prodrugs are formed from a fatty acid carrier and a neuroactive drug which is unable to pass the bbb on its own. A similar system is disclosed in WO89/07938.

Still another approach is the implantation of controlled release polymers which release the active ingredient from a matrix-system directly into the nervous tissue. However, this approach is invasive and requires surgical intervention if implanted directly into the brain or spinal cord [see Sabel *et al.,* U.S. Patent No.4,883,666; and Sabel *et al.,* U.S. Patent Application Serial No. 07/407,930].

To overcome these limitations, another approach has been tried in which drug carrier systems are used such as liposomes, erythrocyte ghosts, antibody-conjugates, and monoclonal antibody conjugates. One of the major problems in targeted drug delivery is the rapid opsonization and uptake of injected carriers by the reticuloendothelial system (RES), especially by the macrophages in the liver and spleen. This obstacle may be partially overcome in the case of liposomes by incorporation of so-called "stealth" lipids, such as phosphatidylinositol, monosialoganglioside, or sulfogalactosylceramide. However, all of these systems lack the versatility to permit a wide-range application in medicine.

### II. Nanoparticles as drug delivery vehicles

Nanoparticles offer new hope for the detection and treatment of several diseases, in particular of cancer. Nanoparticles, thus, hold great potential for many fields of medicine, particularly cancer treatment. The promise of delivering tumor-killing drugs directly to cancerous cells, thus averting the unwanted side-effects of chemotherapy, has generated a lot of interest in nanoparticles among the medical community.

An approach to carry and direct drugs to a desired target tissue is disclosed in WO 95/022963. Therein, a drug targeting system is disclosed comprising nanoparticles made of a polymeric material, said nanoparticles comprising a drug to be delivered to said mammal and a surfactant coating deposited thereon; and a physiologically acceptable carrier and/or diluent allowing the transport of said nanoparticles to the target within the mammal after administration. In this approach, nanoparticles are used comprising polymeric particles having a diameter of preferably < 1,000 nm.

Alkylcyanoacrylates (ACA, C1 to C6) have been proven to be valuable monomers for several applications. Besides the broadly known use as "super glue", they are employed in surgery for wound closure (e.g. Indermil®, n-butylcyanoacrylate BCA). Both applications base on the fact that the anionic polymerization is easily initiated by traces of nucleophiles like water from humidity and dermal water, amines which are present in skin proteins, alcohols or phosphines.

In the last years, considerable efforts have been put in the synthesis of poly(alkylcyanoacrylate) nanoparticles. Poly(alkylcyanoacrylate) nanoparticles in particular are biocompatible, biodegradable and are reported to show a distinct tendency for the adsorption respectively entrapment of bioactive compounds, making them to promising candidates for the use as drug carrier systems. A large number of different compounds has been used as "payload", ranging from inorganic crystallites, e.g. magnetite {Arias, 2001 #1}, to various drugs (methotrexate {Reddy, 2004 #2}, doxorubicin {Steiniger, 2004 #7; Kattan, 1992 #3; Gulyaev, 1999 #11}) and even oligopeptides (dalargin {Alyautdin, 1995 #10}, {Olivier, 1999 #13}) or proteins (insulin {Couvreur, 1988 #27; Behan, 2001 #5; Sullivan, 2004 #8}).

The first process for the formation of poly(alkylcyanoacrylate) (nano)particles has been developed by Couvreur {Couvreur, 1979 #9} in the late 1970s employing a HCl-solution (10⁻² to 10⁻³ mol·l⁻¹) containing a polymeric, non-ionic surfactant as steric stabilizer, to which the alkylcyanoacrylate monomer is added dropwise. Since then a large number of studies (see {Limouzin, 2003 #22}) has reported the application of dispersion and emulsion techniques, with or without surfactant. The described particles show a broad distribution of sizes ranging from under 100 nm to more than 1 µm. Particle size, stability of the dispersion and the molar masses of the polymer depend largely on the pH of the continuous phase {Behan, 2001 #29}, {Lescure, 1992 #30}, {El-Egakey, 1983 #31}, {Douglas, 1984 #32} and on the type and concentration of surfactant {Douglas, 1985 #33}, {Vasnick, 1985 #35}.

Despite the extensive application of the emulsion polymerization with non-ionic or polymeric surfactants for the preparation of poly(alkylcyanoacrylate) nanoparticles, there are several limitations, especially the low polymer content of the dispersions (~ 1 wt%) and the high amount of surfactant compared to the monomer (ratio surfactant to monomer of 1:1 or even more e.g. {Seijo, 1990 #34}, {Alyautdin, 1995 #36}). Additionally, the stabilizer present in the commercially available monomer causes severe problems. Since the applied stabilizers are lewis acids in an unknown concentration (MeSO₃H, SO₂), an influence on the particle properties can be expected and cannot easily be controlled with the techniques applied so far. It has been reported that the amount of SO₂ affects the particle size {Labib, 1991 #16}, {Lescure, 1992 #30} and therefore leads to a lack of reproducibility if using different batches of the alkylcyanoacrylate monomer.

Up to now, modification of the particle surface is achieved by the choice of surfactant, which is physically adsorbed, like e.g. polysorbates {Alyautdin, 1995 #10}, {Olivier, 1999 #13}, or chemically bonded via e.g. a hydroxy group of dextran {Chauvierre, 2004 #18}, {Douglas, 1985 #17} or PEG {Peracchia, 1997 #21} to the particles' surface. The modification with polysorbates allows the particles to permeate through the blood brain barrier, while PEGylated particles show long persistence in the circulatory system.

Nevertheless, further chemical functionalization of the polymer particles to specifically designed surface characteristics is difficult to achieve, because of the occupation of the particle surface by surfactant molecules (due to high amount of surfactant used) and the absence of anchor-groups like COO⁻. These groups are needed in order to conjugate (bio)molecules {Lathia, 2004 #39; Nakajima, 1995 #40; Rasmussen, 1991 #41} via EDC coupling like proteins (e.g. antibodies) for addressing specific receptors for cellular response.

### III. Nanoparticles formed by the miniemulsion method

From the prior art it is also known to carry out conversions to polymers in miniemulsions. Miniemulsions are dispersions of, e.g., water, an oil phase, and one or more surfactants which have a droplet size of from about 50 to 500 nm. The miniemulsions were considered metastable (cf. Emulsion Polymerization and Emulsion Polymers, Editors P. A. Lovell and Mohamed S. El-Aasser, John Wiley and Sons, Chichester, New York, Weinheim, 1997, pages 700 et seq.; Mohamed S. El-Aasser, Advances in Emulsion Polymerization and Latex Technology, 30th Annual Short Course, Volume 3, Jun. 7-11, 1999, Emulsion Polymers Institute, Lehigh University, Bethlehem, Pa., USA). These dispersions find broad application in the art in cleaning products, cosmetics or body care products.

The preparation of aqueous primary dispersions by means of the free-radical miniemulsion polymerization of olefinically unsaturated monomers is known for example from International Patent Application WO 98/02466 or from German Patents DE-A-196 28 143 and DE-A-196 28 142. In the case of these known processes the monomers can be copolymerized in the presence of different low molecular mass, oligomeric or polymeric hydrophobic substances.

From WO 2006/029845 a method of preparing nanoparticles is known containing the steps of:
- providing a reaction system comprising O and W type liquid phases, a stabilizer, one or more pharmaceutical agents and polymerizable monomers,
- forming an O/W type miniemulsion, and
- polymerizing said monomers in order to form nanoparticles.

WO 2006/029845 in particular makes use of the miniemulsion method as disclosed in K. Landfester, "Polyreactions in miniemulsions", Macromol. Rapid Comm. 2001, 896-936. K. Landfester, N. Bechthold, F. Tiarks, and M. Antonietti, "Formulation and stability mechanisms of polymerizable miniemulsions". This technology basically differs from the well known conventional methods by involving a different order of manufacturing steps: in this approach, a miniemulsion, containing dispersed hydrophobic, monomer containing droplets in a hydrophilic continuous phase, are formed to polymeric particles. In the conventional approach, the polymers are directly formed from the solution containing monomers.

However, WO 2006/029845 does not disclose that the polymerization is initiated by one or more (primary) amines.

WO94/020106 discloses hydrophilic polyglutamate microcapsules having a diameter of 1-250 µm useful for in vivo distribution. The microcapsules are intended for specific targeting to precisely defined tissues. However, WO94/020106 does not make use of the miniemulsion method.

WO 03/026590 is related to compositions and methods for selective binding of amino acid oligomers to semiconductor and elemental carbon-containing materials. One form is a method for controlling the particle size of the semiconductor or elemental carbon-containing material by interacting an amino acid oligomer that specifically binds the material with solutions that can result in the formation of the material. The same method can be used to control the aspect ratio of the nanocrystal particles of the semiconductor material. Also here, however, it is not disclosed to form nanoparticles by the minimemulsion method.

From "Synthesis of Nanosize Silica in a Nonionic Water-in-Oil Microemulsion: Effects of the Water/Surfactant Molar Ratio and Ammonia Concentration", Journal of Colloid and Interface Science 211, 210-220 (1999), it is known to add ammonia to a microemulsion However, this publication describes the production (by polycondensation) of silica nanoparticles by a variation of the so-called Stöber process. Additionally a non-ionic surfactant and cyclohexane were added and yielded nanosized silica in the range of 30-70 nm. It is noted that at small surfactant concentrations, the particle size increases when the ammonia concentration increases. At higher surfactant concentrations, a minimum in particle size occurs as the ammonia concentration increased. Apart thereform, this citation does not disclose the use of the miniemulsion method.

### Objects of the invention

It is an object of the present invention to provide a method of producing nanoparticles, wherein the amount of surfactants is considerably lowered and functional groups may be introduced.

It is also an object of the present invention to provide an improved method for the manufacture of PACA nanoparticles, which particles can be obtained in high yield, solid content, and having a small particle size and a narrow, uniform particle distribution.

It is a further object of the invention to provide a delivery vehicle for a pharmaceutical agent which can be specifically adapted to desired release characteristics. It is in particular an object of the invention to provide a delivery vehicle which is suitable to be used for varying medical conditions and which is able to specifically cross the major physiological or biological barriers of the animal or human body and which subsequently is showing modified release characteristics such as sustained release or prolonged release of the drug in the target tissue.

Furthermore, it is an object of the present invention to provide a method for the manufacture of drug delivery vehicles, which is suitable for the large-scale production of said vehicles.

### Summary of the invention

These objects are achieved by the subject-matter of the independent claims. Preferred embodiments are set forth in the dependent claims.

It turned out that the initiation of the polymerzation step of the miniemulsion method with primary or secondary amines (primary amines are defined as also including ammonia in this invention) on the one hand leads to small PACA nanoparticles having a narrow size distribution and, on the other hand, leads to the possibility to functionalize the nanoparticles.

A two step miniemulsion process has been applied for the preparation of PACA nanoparticles, preferably poly(n-butylcyanoacrylate) (PBCA) nanoparticles. As an example, in the first step, a miniemulsion is prepared from n-butylcyanoacrylate in hydrochloric acid solution using sodium dodecyl sulfate as surfactant. In the second step, a base solution is added to initiate polymerization and the polymeric particles are formed. Using primary or secondary amines or amino acids as initiators allowed the convenient functionalization of the polymer particles' surface. It is noted that it further turned out that also tertiary or quaternary amines can work in the context of the present invention as long as they are capable as acting as a nucleophile. However, primary and secondary amines are preferred in the present invention.

The influence of surfactant concentration and sonication time on particle size and size distribution has been studied as well as the influence of pH, concentration and amount of initiator on the particle size and the distribution of the molar mass of the polymer. The detected pH-dependence of the particle's ζ-potential indicates the presence of carboxyl groups on the particles' surface after the initiation with amino acids.

As a result, the invention discloses the preparation of reproducibly functionalizable and loadable PACA nanoparticles, in particular PBCA nanoparticles, with a narrow size distribution which are stable in a dispersion. The nanoparticles can be prepared in such a way that with a solid content higher than 10 wt% using a minimum of the anionic surfactant sodium dodecyl sulfate (SDS) is achieved.

A very convenient way to meet these requirements is the application of the miniemulsion technique in which the polymerization is initiated after a stable n-butylcyanoacrylate miniemulsion in water has been formed as it was already shown in one single model experiment using the miniemulsion technique in order to create poly(alkylcyanoacrylate) nanoparticles with a solid content of 5% {Limouzin, 2003 #22}.

Taking advantage of the high stability of miniemulsions obtained by a hydrophobic agent in order to prevent Ostwald ripening, it will be shown that it is possible to increase the amount of dispersed BCA-monomer even further and therefore the solid content of the final dispersion to more than 10%. The polymerization is initiated in the simplest case by the addition of a hydroxide solution. The influence of surfactant concentration and sonication time on particle size and size distribution has been studied as well as the influence of pH, concentration and amount of initiator solutions on the particle size and the distribution of the molar mass of the polymer. It will be shown that the application of mono- or multifunctional amines as initiator allows the introduction of functional groups to the polymer {Kulkarni, 1971 #43}, {Leonard, 1966 #44}, {Pepper, 1978 #42} and thus to the particle. The detected pH-dependence of the particle's ζ-potential indicates the presence of carboxyl groups on the particles' surface after the initiation with amino acids.

### Detailed description of the invention

According to a first aspect, the present invention provides a method of producing nanoparticles comprising the steps of:
a) preparing an O/W miniemulsion, comprising O and W (aqueous) type liquid phases, one or more stabilizers, and polymerizable ACA monomers,
b) polymerizing said monomers by anionic polymerization, and isolating the produced nanoparticles,
characterized in that
the polymerization in step b) is initiated by one or more primary or secondary amines.

Surprisingly, it turned out that the addition of primary or secondary amines for initiating the polymerization of the monomers is leading to a narrow and uniform size distribution of said nanoparticles and led to a significantly reduced amount of surfactants needed in the manufacture of said nanoparticles. As also mentioned above, the invention is not necessarily restricted to the use of primary or secondary amines, and also tertiary, quaternary amines and other reagents might be used as long as they are strong nucleophiles. The advantages of this development are evident: the smaller the nanoparticles are, the better they will be suitable for the envisioned applications, in particular for *in* vivo applications for crossing the blood brain barrier. Furthermore, the reduced amount of surfactant (or stabilizer) needed for preparing the nanoparticles will also reduce the in vivo load of the surfactant for the patient and will allow chemical functionalization of the polymer particles to specifically designed surface characteristics, because of the occupation of the particle surface by surfactant molecules is avoided. The advantages will be outlined in more detail in chapter "Examples".

The term "nanoparticles" as used herein generally denotes a carrier structure which is biocompatible and sufficiently resistant to chemical and/or physical destruction by the environment of use such that a sufficient amount of the nanoparticles remain substantially intact after entry into the human or animal body following intraperitoneal or oral or intravenous administration so as to be able to reach the desired target organ or tissue, e. g. the brain, the liver, the kidneys, the lungs etc.

According to a preferred embodiment, said nanoparticles have a diameter of between 1 nm and 20 µm, preferably between 10 nm and 10 µm and most preferably between 50 nm and 1,000 nm.

Furthermore, the nanoparticles as defined above preferably comprise a surfactant coating deposited thereon.

A treatment of the nanoparticles with a sufficient coating of an appropriate surfactant allows the adsorbed drug to better traverse physiological barriers as the bbb. Reference is made to several documents disclosing this effect, in particular to WO 95/22963, which is incorporated herein in its entirety.

The amount of the one or more stabilizers is between 1 and 50 %, preferably between 5 and 30% and most preferably between 10 and 25%wt.-% based on the monomer phase. It should be further noted that the "amount of stabilizer" as used herein also includes the overall amount of several combined stabilizers. For example, said amount of between 10 and 25% by weight might be composed of 10% of sodium dodecylsulfate and 10% Polysorbate 80

The term "stabilizer" as used in this invention shall comprise any substance capable of stabilizing an emulsion. Those substances are preferably surface active and/or amphiphilic substances, i.e. surfactants.

The surfactant coating and/or the stabilizer preferably comprises one or more of the following substances:
fatty acid esters of glycerols, sorbitol and other mono- or multifunctional alcohols, preferably benzyl alcohol, glycerol monostearate, sorbitan monolaurate, or sorbitan monoleate; phospholipids, phosphoric acid esters, polysaccharide, benzyl benzoate, polyethylene glycol (PEG 200, 300, 400, 500, 600), polyethylene glycol hydroxystearate, preferably Solutol HS 15; poloxamines, preferably poloxamine 904, 908 or 1508; polyoxyethylene ethers and polyoxyethylene esters; ethoxylated triglycerides; ethoxylated phenols and ethoxylated diphenols; surfactants of the Genapol TM and Bauki series; polyoxyl castor oils, preferably Cremophor ELP; lecithin, metal salts of fatty acids, metal salts of fatty alcohol sulfates; and metal salts of sulfosuccinates; preferably polysorbates, more preferably polysorbate 20, 60 and most preferably polysorbate 80; preferably poloxamers, more preferably poloxamer 188, 338 or 407; preferably polyoxyethylene glycols, more preferably Lutensol 50 or 80; anionic surfactants as they are known in the art (see, for example, M.J. Rosen: "Surfactants and interfacial phenomena", Wiley & Sons, Inc. New York Chichester Brisbane Toronto Singapore 1989), e.g. sodium dodecyl sulfate; and mixtures of two or more of said substances.

As indicated above, polysorbate 80 is most preferred.

It is noted that, if a surfactant coating is present, the present invention also provides a method, wherein the coating is at least partially removed from the obtained nanoparticles. This can preferably be done by dialysis or centrifugation. It surprisingly turned out that, although required in the method of manufacture per se, the stabilizers may be at least partially removed after the final nanoparticles were formed. In other words, an "excess" of stabilizers can be removed which is not required for maintaining the stability of the nanoparticles, but can cause potential risks for *in vivo* applications. It is assumed that the lowest possible amount of stabilizers in the nanoparticles should be regarded as having the lowest *in vivo* risk.

A preferred method of polymerisation is the miniemulsion technique as developed by K. Landfester et al.

Miniemulsions are dispersions of critically stabilized oil droplets with a size between 50 and 500 nm prepared by shearing a system containing oil, water, a surfactant and a hydrophobe. Polymerizations in such miniemulsions result in particles which have about the same size as the initial droplets (K. Landfester, Polyreactions in miniemulsions, Macromol. Rapid Comm. 2001, 896-936. K. Landfester, N. Bechthold, F. Tiarks, and M. Antonietti, Formulation and stability mechanisms of polymerizable miniemulsions. Macromolecules 1999, 32, 5222. K. Landfester, Recent Developments in Miniemulsions - Formation and Stability Mechanisms. Macromol. Symp. 2000,150,171).

As mentioned above, the already known miniemulsion technique as, for example, disclosed in Landfester et al. (supra), may be used to prepare the miniemulsion. This may, for example be done by at first combining monomers in an O phase and a W phase and a stabilizer (defined above). It is noted that the aqueous phase may contain further ingredients, for example HCl for setting a suitable pH value. Second, the reaction system may be mixed by, for example, a homogenizer or the like, in order to mix all ingredients.

The preparation of the miniemulsion itself is performed by applying high shear forces to the reaction system, for example by ultrasound and high pressure homogenizers. Furthermore, the shear forces may be applied for a time range of for example from 1-10 min depending on the size of the reaction system and the homogenizer used Basically, a time range of between 2 and 4 min is regarded as being sufficient. However, it is noted that the details and conditions of preparing nanoparticles may vary depending on many factors, as for example the precise equipment used etc.

The ultrasound homogenizer may have an amplitude of about 60-100%, preferably about 70-90%.

According to an embodiment, the temperatures used in this process are preferably from -1 to 5 °C, preferably 0 °C. However, the temperature range is not restricted thereto and wider ranges can be used.

Generally the weight ratio of O to W phase is from 5-40 % w/w, preferably 20-30 % w/w and more preferably about 25 % w/w.

According to a preferred embodiment, the amine is selected from the group consisting of ammonia, tris-base, or from amino acids, preferably phenylalanine, glycine or 6-aminohexanoic acid. The latter turned out to bring about advantageous results, however, other compounds selected from the group of amines may also be used. It is noted that the effects of the present invention as explained above, can not be used by the conventional approach of using NaOH alone.

In the method of the present invention, perferably one or more pharmaceutical agents are contained in the W and/or in the O phase, preferably selected from a therapeutic agent and a diagnostic agent.

It is noted that the terms "drug" and "therapeutic agent" are used interchangeably herein.

The therapeutic agent is preferably selected from substances which are incapable or not sufficiently capable of crossing physiological barriers without a delivery vehicle or carrier, wherein the physiological barrier preferably is selected from the group consisting of blood-brain barrier (bbb), blood-air barrier, blood-cerebrospinal fluid barrier and buccal mucosa.

According to a preferred embodiment, the delivery vehicle of the invention comprises one or more therapeutic agents selected from the group consisting of drugs acting at synaptic sites and neuroeffector junctional sites; general and local analgetics; hypnotics and sedatives; drugs for the treatment of psychiatric disorders such as depression and schizophrenia; anti-epileptics and anticonvulsants; drugs for the treatment of Parkinson's and Huntington's disease, aging and Alzheimer's disease; anti-obesity drugs; excitatory amino acid antagonists, neurotrophic factors and neuroregenerative agents; trophic factors; drugs aimed at the treatment of CNS trauma or stroke; drugs for the treatment of addiction and drug abuse; antacoids and anti-inflammatory drugs; chemotherapeutic agents for parasitic infections and diseases caused by microbes; immunosuppressive agents and anti-cancer drugs; vitamines; hormones and hormone antagonists; heavy metals and heavy metal antagonists; antagonists for non-metallic toxic agents; cytostatic agents for the treatment of cancer, preferably doxorubicin; diagnostic substances for use in nuclear medicine; immunoactive and immunoreactive agents; transmitters and their respective receptor agonists and receptor antagonists, their respective precursors and metabolites; transporter inhibitors; antibiotics; antispasmodics; antihistamines; antinauseants; relaxants; stimulants; sense and antisense oligonucleotides; cerebral dilators; psychotropics; antimanics; vascular dilators and constrictors; anti-hypertensives; drugs for migraine treatment; hypnotics, hyperglycemic and hypoglycemic agents; anti-asthmatics; antiviral agents, preferably anti HIV agents; genetic material suitable for the DNA or antisense treatment of diseases; and mixtures thereof. Regarding the addition of such agents in the above mentioned method of preparing nanoparticles it is noted that it might be preferred to add hydrophobic agents already before the polymerisation is started, wherein hydrophilic agents might be added before or following that step (by adsorption to the nanoparticle's surface).

The term "DNA" as used in the present specification basically refers to any DNA conceivable in the present field of the art. In preferred embodiments, the term "DNA" is meant to comprise two types of DNA, i. e. plasmid DNA, more preferably plasmid DNA comprising the information of tumor suppressor genes, even more preferably plasmid DNA comprising the information of the tumor suppressor genes p53 and pRB, on the one hand, and antisense oligonucleotides, more preferably antisense oligonucleotides against oncogenes, even more preferably antisense oligonucleotides against oncogenes like Bcl2, on the other hand. There may be used one type of DNA (and, consequently, one type of DNA-loaded nanoparticles) in the present invention. Alternatively, two or more types of DNA may be used, resulting into a plurality of types of nanoparticles loaded with different types of DNA and useable in accordance with the present invention.

Surprisingly, DNA and particularly DNA of the above two types could be adsorbed onto nanoparticles, and the resulting DNA-nanoparticle complexes could be inoculated into the organism, particularly into the organism suffering from cancer (specifically, but not limited to brain cancer). Thereafter, a suppression of the tumor proliferation could be observed, and even a tumor necrosis and apoptosis could be induced.

In a preferred, but not essential embodiment of the invention, plasmid DNA comprising a promoter, more preferably plasmid DNA comprising an inducible promoter, can be loaded onto the nanoparticles. By the novel step to load the DNA containing an inducible promoter onto the nanoparticle and to express it within the cell, an inducible promoter, and thereby an external control of the expression of the relevant gene may be achieved, and the gene may be "switched" on and off at will. As an unexpected advantage over the prior art, the timing of the gene/DNA expression can be controlled. Such a control may reduce toxic side effects of a continuous gene expression and/or may lower the probability that cells become resistant to the gene products, producing a negative selection.

In a preferred embodiment of the invention, the human papilloma virus upstream regulatory region (HPV-URR) was used as the inducible promoter. The expression of the tumor suppressor is induced after the administration of Dexamethasone or other inducers or compounds. In that way, an apoptosis of the tumor cells as well as a regression of the tumor could be achieved. Other exemplary promoters useable in accordance with the present invention are the cytomegalia virus (CMV) promoter or the simian virus 40 (SV 40) promoter.

Furthermore, strong effects including a tumor regression could be achieved by a combination administration of one or more than one type(s) of DNA-containing nanoparticles and nanoparticles containing one or more than one cytostatically effective substance(s).

In a preferred embodiment, tumor suppressor DNA, even more preferred behind an inducible promoter, may be injected prior to inoculation of a nanoparticle complex containing a cytostatically effective compound. In an even more preferred embodiment, the cytostatically effective compound is doxorubicine.

In the process of transfection of DNA into cells, and in the process of administration of a DNA to a target organ in the human or animal body as well, the first step comprises the preparation of nanoparticles in a way defined above.

For further information it is explicitely referred to WO 2004/017945, and its content is incorporated herein in its entirety.

Typical active ingredients (e.g., drugs) can be any substance affecting the nervous system or used for diagnostic tests of the nervous system. These are described by Gilman et al. (1990), "Goodman and Gilman's - The Pharmacological Basis of Therapeutics", Pergamon Press, New York, and include the following agents:
acetylcholine and synthetic choline esters, naturally occurring cholinomimetic alkaloids and their synthetic congeners, anticholinesterase agents, ganglionic stimulants, atropine, scopolamine and related antimuscarinic drugs, catecholamines and sympathomimetic drugs, such as epinephrine, norepinephrine and dopamine, adrenergic agonists, adrenergic receptor antagonists, transmitters such as GABA, glycine, glutamate, acetylcholine, dopamine, 5-hydroxytryptamine, and histamine, neuroactive peptides; analgesics and anesthetics such as opioid analgesics and antagonists; preanesthetic and anesthetic medications such as benzodiazepines, barbiturates, antihistamines, phenothiazines and butylphenones; opioids; antiemetics; anticholinergic drugs such as atropine, scopolamine or glycopyrrolate; cocaine; chloral derivatives; ethchlorvynol; glutethimide; methyprylon; meprobamate; paraldehyde; disulfiram; morphine, fentanyl and naloxone; psychiatric drugs such as phenothiazines, thioxanthenes and other heterocyclic compounds (e.g., halperiodol); tricyclic antidepressants such as desimipramine and imipramine; atypical antidepressants (e.g., fluoxetine and trazodone), monoamine oxidase inhibitors such as isocarboxazid; lithium salts; anxiolytics such as chlordiazepoxyd and diazepam; anti-epileptics including hydantoins, anticonvulsant barbiturates, iminostilbines (such as carbamazepine), succinimides, valproic acid, oxazolidinediones and benzodiazepines, anti-Parkinson drugs such as L-DOPA/CARBIDOPA, apomorphine, amatadine, ergolines, selegeline, ropinorole, bromocriptine mesylate and anticholinergic agents; antispasticity agents such as baclofen, diazepam and dantrolene; neuroprotective agents, such as excitatory amino acid antagonists, neurotrophic factors and brain derived neurotrophic factor, ciliary neurotrophic factor, or nerve growth factor; neurotrophine (NT) 3 (NT3); NT4 and NT5; gangliosides; neuroregenerative agents; drugs for the treatment of addiction and drug abuse include opioid antagonists and antidepressants; autocoids and anti-inflammatory drugs such as histamine, bradykinin, kailidin and their respective agonists and antagonists; chemotherapeutic agents for parasitic infections and microbial diseases; anti-cancer drugs including alkylating agents (e.g., nitrosoureas) and antimetabolites; nitrogen mustards, ethylenamines and methylmelamines; alkylsulfonates; folic acid analogs; pyrimidine analogs, purine analogs, vinca alkaloids; and antibiotics.

It is emphasized again that the most preferred pharmaceutical agent for use in the present invention is doxorubicine represented by the following formula:

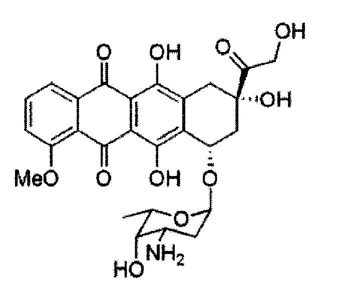

According to a preferred embodiment, the diagnostic agent is selected from the group consisting of diagnostics useful in the diagnosis in nuclear medicine and in radiation therapy.

In a preferred embodiment, the O phase comprises a lipophilic solvent or hydrophobe, preferably n-hexane, hexadecane, liquid paraffin, vitamine E, miglyol or fatty acid esters of triglycerides. The amount of hydrophobe is usually relatively small and should be sufficient to prevent Ostwald ripening (about 2-20% w/w based on the overall weight of the O phase (further containing the monomer)).

Preferably, the one or more pharmaceutical agents and the monomers are contained in the O phase. In this case, the pharmaceutical agents are finely distributed into the nanoparticles formed by the polymerisation step. As an alternative, the one or more pharmaceutical agents are present in the W phase and the monomers are contained in the O phase. It turned out that also in this case, a remarkable amount of the pharmaceutical agent is incorporated into the nanoparticles formed. We do not want to be bound to any specific theory, however, it is assumed that the pharmaceutical agents contained in the W phase are intimately contacted with the O phase by preparing the miniemulsion, for example by applying high shear forces, leading to a distribution of the agent also in the O phase.

Additionally, solution mediators may be used to bring agents into solution, which under normal circumstances would not dissolve in a sufficient amount in the solvent. Examples for this group of substances are dimethylsulfoxide, dimethylformamide, polysorbate 80, vitamine E, polyethylene glycol (PEG 200, 300, 400, 500, 600), poloxamers, polyoxyl castor oils (Cremophor ELP), polyoxyethylene glycols, polyethylene glycol hydroxystearate (Solutol), Labrafil, Labrasol, lecithin, propylene glycole, benzyl benzoate, glycerol or fatty acid esters of mono- or multifunctional alcohols or triglycerides.

It is furthermore possible to add the one or more pharmaceutical agents to both, the O and the W phase, if this is desired.

In a preferred embodiment, the stabilizer used is contained in the W phase. The W phase preferably is water or an aqueous solution containing acids, preferably hydrochloric or phosphoric acid. The polymerizable monomers are used in order to form a polymeric material, which preferably is selected from the group consisting of polycyanoacrylates, preferably polyalkylcyanoacrylates, and derivatives, copolymers and mixtures thereof. The term "polyalkylcyanoacrylate" is defined herein as preferably comprising an alkyl group of C1-C6.

The polymeric materials used (produced) in the present invention preferably are biodegradable. This terms denotes any polymeric material which is known as being suitable for uses in the body of a living being, i.e., is biologically inert and physiologically acceptable, non-toxic, and, in the delivery systems of the present invention, is biodegradable in the environment of use, i.e., can be resorbed by the body.

According to a second aspect, the invention is directed to nanoparticles obtainable by the method as defined above and a pharmaceutical composition, comprising those nanoparticles and a pharmaceutically acceptable carrier and/or diluent.

In order to use the nanoparticles in a practical embodiment, they may be reconstituted into a suspension with distilled water or normal saline at physiological pH and osmolarity.

Typically, the nanoparticles are present in the injectable suspension at a concentration ranging from 0.1 mg nanoparticles per ml suspending fluid to 100 mg nanoparticles per ml suspending fluid. 10 mg nanoparticles per ml is preferred. The amount of nanoparticles used will strongly depend on the amount of pharmaceutical agent contained in an individual nanoparticle and the skilled artisan or the physician in charge will be readily able to adapt the dosage of the nanoparticles to the specific circumstances.

Preferably, the delivery vehicle or the pharmaceutical composition are showing a prolonged release or sustained release of said one or more pharmaceutical agents *in vivo.* Those nanoparticles made in accordance with the principles of the invention biodegrade in periods of time ranging from a few hours to 6 months or more.

Alternatively, the pharmaceutical composition may take other forms required to transfer the delivery vehicle of the invention to and across other physiological barriers, for example to and across the blood-air barrier. Then it may, for example have the form of an aerosol or the like in order to deliver the composition by inhalation to the barrier in question.

The present invention further provides the use of the delivery vehicle or a pharmaceutical composition as defined herein for the manufacture of a medicament for the treatment of diseases and conditions, requiring a pharmaceutical agent to cross one or more physiological barriers. It is in particular used for the treatment of diseases corresponding to the drugs listed hereinabove.

In particular, the delivery vehicle will find application in the treatment of diseases related to the CNS. Furthermore, this includes the treatment of AIDS, since in many people with advanced AIDS - possibly a third of adults and half of all children - HIV also infiltrates and harms the brain, triggering HIV-associated dementia. The disorder is marked by poor concentration, decreased memory and slow thinking and movements. However, it is particularly hard to target the virus in the brain. Here, the present invention may open up new therapeutic successes by delivering anti-HIV agents to the brain.

All publications, patent applications, patents, and other references mentioned herein are incorporated by reference in their entirety. In case of conflict, the present specification, including definitions, will control. In addition, the materials, methods, and examples are illustrative only and not intended to be limiting.

### Brief description of the drawings

Figure 1: Evolution of particle size (open circles) and *M*_{w} with concentration of surfactant for a sonication time of 150 s (dotted lines are guides for the eye).
Figure 2: GPC elugram obtained from a BCA miniemulsion and evolution of elution time obtained from a PBCA dispersion during the course of four weeks.
Figure 3: TEM-Images of dispersions prepared with SDS (sample S-10), Lutensol AT50 (sample L-10) and Tween 20 (sample T-10) (pictures left to right); the images of the dispersions prepared with Lutensol AT50 and Tween 20 could only be obtained after redispersing the particles.
Figure 4: Evolution of pH compared with the calculated value. The volumes of the respective NaOH-solutions were chosen to yield the calculated pH for the final dispersions. The consumption of OH- during the reaction has been neglected in the calculation (dotted lines are guides for the eye).
Figure 5: Molar mass evolution of polymer obtained with initiator (NaOH-solution) volume (number given on the right side of each slice), particles frozen after seven days.
Figure 6: Proposed mechanism of de/repolymerization. {Ryan, 1996 #37}
Figure 7: Evolution of elution volume with initiator (ammonia solution) volume (given on the right side of each slice). Samples frozen two days after preparation.
Figure 8: Evolution of elution volume with initiator (tris base solution) volume (given on the right side of each slice). Samples frozen two days after preparation.
Figure 9: Evolution of particle size, initiated with 0.5 mol 1⁻¹6-aminohexanoic acid (left) and initiated with glycine solutions (right) (dotted lines are guides for the eye). The numbers in the legend of the right diagram indicate the molar concentration (before dash) and the pH of the solution (after dash).
Figure 10: GPC elution volumes of 6-aminohexanoic acid initiated samples: a) pH 4.4 b) pH 5.5 c) pH10.
Figure 11: GPC elution volumes of glycine initiated samples: a) Gly 0.1 pH3.4; b) Gly 0.5, pH 3.4; c) Gly 2.0, pH 3.4.
Figure 12: ζ-potential at pH 3 and 10 before and after dialysis.
Figure 13: ¹H-NMR-spectrum of NaOH-solution (0.1 mol l⁻¹, calculated pH 7) initiated polymer.
Figure 14: ¹H-NMR spectrum of 6-aminohexanoic acid-solution (pH4.4, 0.5 mol l⁻¹) initiated polymer.

### Examples

### Materials

n-Butylcyanoacrylate (BCA, Indermil^{®}, Henkel Loctide) was used as received. Hydrochloric acid (0.1 mol l⁻¹), sodium hydroxide solution (0.1 mol l⁻¹), tris-base (tris-(hydroxymethyl)-aminomethane), ammonia-solution (25%), sodium dodecyl sulfate (SDS) and all amino acids (6-aminohexanoic acid, arginine (Arg), aspartic acid (Asp), glutamic acid (Glu), cysteine (Cys), glycine (Gly), lysine (Lys)) were purchased from Merck, except phenylalanine (Phe) which was purchased from Aldrich. Tween 20 was purchased from Sigma-Aldrich. Lutensol AT50, a poly(ethyleneoxide)-hexadecyl ether with an EO block length of about 50 units, was received as a gift from BASF AG. All chemicals were used as received.

### Synthesis of nanoparticles

### Standard procedure for the preparation of a n-butylcyanoacrylate miniemulsion and subsequent initiation of the polymerization

A solution of 0.3 g of SDS in 12.0 g of hydrochloric acid (0.1 mol·l⁻¹) was added just prior to ultrasonication to a solution of 0.125 g of hexadecane in 3.0 g BCA. The two-phase mixture was sonified with a Branson sonifier W450 (90% amplitude 0.5 inch tip) for 2.5 min under ice cooling. After sonication a milky white emulsion is obtained. The amounts used in the standard procedure are varied, the ratio of the reactants used is maintained. The polymerization was initiated by pouring the miniemulsion into a sodium hydroxide solution (0.1 mol·l⁻¹) under stirring.

### Variation of surfactant

In order to determine the influence of the type of surfactant, the miniemulsion was prepared in the way described above but with different surfactants. Besides SDS, Lutensol AT 50 and Tween 20 were used (see Table 1). The polymerization was initiated by pouring the miniemulsion into 12.0 g of sodium hydroxide solution (0.1 mol·l⁻¹) under stirring.

### Variation of sonication time and surfactant amount

The miniemulsion was prepared as described above with SDS as surfactant. After a sonication time of 90, 120, 150, and 180 s 500 µl of the miniemulsion were withdrawn and injected into 375 µl of sodium hydroxide solution (0.1 mol·l⁻¹).

### Time dependence of molar mass

A miniemulsion was prepared in the way described above (BCA 9.0 g, HD 0.375 g, HCl 36.0 g; SDS 0.9 g). A 500 µl sample was pipetted and injected into a vessel immersed in liquid nitrogen just prior to pouring the miniemulsion in 36.0 g of sodium hydroxide solution (0.1 mol·l⁻¹) under stirring. During the first 120 s, each 10 s a 500 µl sample has been taken and treated the same way as described above. This procedure, with longer intervals between the sampling has been carried out over two weeks. The frozen samples were freeze dried. The molar masses of the resulting polymer powder were determined by GPC.

### Variation of type of initiator and amount of initiator

In order to initiate the polymerization, 500 µl of the freshly prepared miniemulsion was injected in one shot into various amounts of sodium hydroxide solution (0.1 mol-1⁻¹, see Table 2), tris-base solution (0.1 mol-1⁻¹), ammonia solution (0.1 mol-1⁻¹) (both Table 3) and various solutions of amino acids (see Table 4).

### Dialysis

The dispersions have been dialysed using Amicon Ultra centrifuge filters (30,000 membrane, Millipore).

### Characterization

The particle size and the ζ-potential were determined with a Zetasizer Nano ZS. For the photon correlation spectroscopy (PCS) measurements 35 µl of the dispersion was pipetted into a single use polystyrene cuvette and diluted with 1.5 ml of demineralised water.

For ζ-potential measurements, 50 µl of the dispersion were diluted to a total volume of 5 ml and the desired pH. The pH was adjusted with 0.1 mol 1⁻¹ NaOH- and HCl-solution.

Gel permeation chromatography (GPC) was used to determine the weight average molecular weight of the poly(n-butylcyanoacrylate) of the nanoparticles. After the polymerization had been completed, the samples were frozen at -22 °C and subsequently freeze-dried. The resulting powders were dissolved in 1 ml of THF, the solution filtered through a 0.45 µm syringe filter. The setup consisted of a Thermal Separations Products P2000 pump with Waters Styragel 5 µm particles, 100 nm pore size, PSS SDV 5 µm particles, 1 µm pore size, PSS SDV 10 µm pore size columns and a Thermal Separations Products AS100 autosampler. The eluent was THF p.a. with a flow rate of 1 ml·min⁻¹. The signal was detected with a Waters 2410 RI-detector and with a Knauer Variable Wavelength Monitor UV-detector. The molar masses were calculated with respect to a PS standard.

The TEM images were obtained using a Philipps TEM 400 with an acceleration current of 80 kV. 5 µl of the dispersion was diluted with 5 ml of demineralized water, a 4 µl drop was put on a carbon coated copper grid (200 mesh) and air-dried. No further staining has been applied.

Up to now, PBCA nanoparticles were mostly prepared by typical emulsion processes. We prepared now stable monomer droplets of the hydrophobic BCA by miniemulsification in 0.1 mol·l⁻¹ hydrochloric acid as continuous phase since strong acids are known to inhibit polymerization of ACAs efficiently {Pepper, 1978 #42}, {Pepper, 1983 #46}, {Costa, 1983 #183}. Then, after creation of the droplets, the anionic polymerization was initiated by pouring the miniemulsion into a NaOH-solution. Evaporation of the monomer during the miniemulsion preparation and heating of the dispersion while polymerization can be minimized by using pulsed ultrasound and cooling the system.

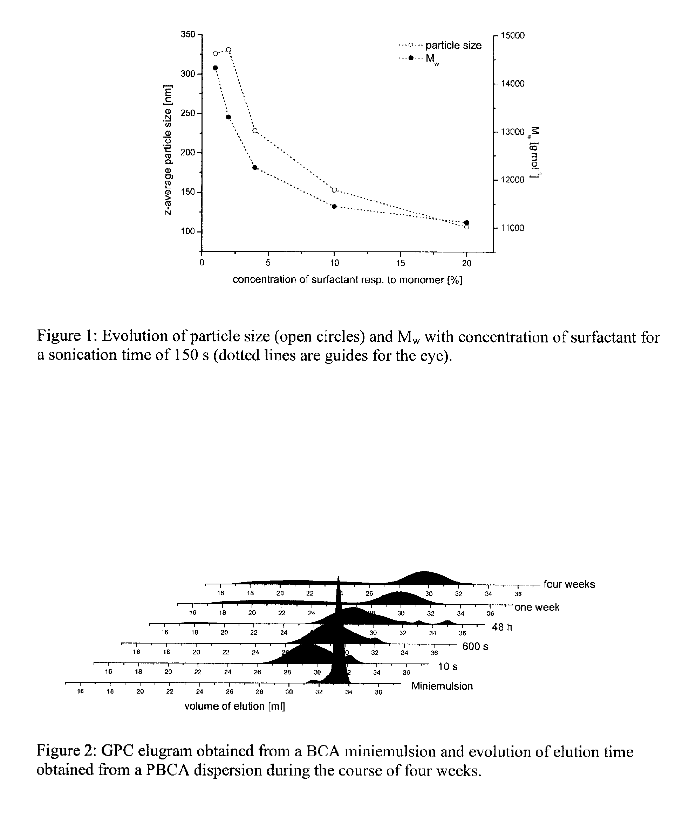

The anionic surfactant SDS has been the first choice for the stabilization of the BCA miniemulsion and the subsequently formed PBCA dispersion. The results are summarized in Table 1. The use of SDS allowed the formulation of PBCA dispersions with a solid content of 10% using 1% of surfactant with respect to BCA leading to stable particles of about 300 nm in diameter. With increasing SDS concentration, the particle size decreases (see also Figure 1) and the size distribution (PDI index) narrows.

**Table 1: Samples with varying SDS amounts and sonication times.**

| Amount of surfactant [wt.% with respect to BCA] | Sonication time [s] | Particle size (Z-average) [nm] | PDl | *M*_{w} [g mol⁻¹] |
|---|---|---|---|---|
| 1 | 90 | 292 | 0.433 | 15,400 |
| 1 | 120 | 358 | 0.308 | 14,800 |
| 1 | 150 | 326 | 0.383 | 14,300 |
| 1 | 180 | 302 | 0.338 | 18,000 |
| 2 | 90 | 295 | 0.289 | 13,300 |
| 2 | 120 | 263 | 0.297 | 14,400 |
| 2 | 150 | 331 | 0.357 | 13,200 |
| 2 | 180 | 292 | 0.297 | 15,100 |
| 4 | 90 | 217 | 0.272 | 14,900 |
| 4 | 120 | 284 | 0.432 | 14,000 |
| 4 | 150 | 228 | 0.346 | 12,200 |
| 4 | 180 | 224 | 0.242 | 12,000 |
| 10 | 90 | 161 | 0.221 | 14,000 |
| 10 | 120 | 161 | 0.250 | 12,400 |
| 10 | 150 | 154 | 0.222 | 11,400 |
| 10 | 180 | 158 | 0.151 | 10,800 |
| 20 | 90 | 155 | 0.272 | 12,600 |
| 20 | 120 | 190 | 0.391 | 10,500 |
| 20 | 150 | 107 | 0.274 | 11,100 |
| 20 | 180 | 183 | 0.205 | 10,300 |

### Figure 1: Evolution of particle size (open circles) and M_{w} with concentration of surfactant for a sonication time of 150 s (dotted lines are guides for the eye).

The equilibrium size of the droplets in miniemulsions is determined by the amount of surfactant with respect to the amount of dispersed phase. This equilibrium is reached by the application of strong shear forces like ultrasound. After a characteristic sonication time, the droplet size cannot be reduced any further. The size distribution still shows a slight narrowing applying a longer sonication time. Figure 1 shows the evolution of the particle size for the sonication time of 150 s representatively for the other sonication times.

The weight average molecular weight *M*_{w} of the polymer (with 1% of SDS) measured two days after mixing with the NaOH solution shows a monomodal distribution at about 15,000 g mol⁻¹. With increasing amount of surfactant, the values for *M*_{w} decrease. This means that after two days the smaller particles are composed of polymer with shorter chains than the larger particles. It has to be noted that the dispersions were frozen two days after preparation. Further experiments show that the molar masses of the polymers change over the course of days until an equilibrium distribution is reached. Samples frozen after one week show no longer monomodal mass distribution but the appearance of long chain polymer (see Figure 2 and discussion below).

All polymer dispersions prepared with SDS showed long-term stability. Even two months after the preparation, no phase separation could be observed. The application of cationic surfactants, namely quaternary amines, led to immediate polymerization when the monomer and the aqueous phase were mixed. Residual primary and secondary amines may be the cause for this.

As non-ionic surfactants Lutensol AT50 and Tween 20 were chosen yielding in unstable dispersions (samples L-10 (sonication time = 120 s, d = 908 nm) and T-10 (sonication time = 120 s, d = 769 nm), respectively) at the desired high solid contents of 10%. Even higher amounts of these surfactants of more than 10% were not able to stabilize the latex dispersions efficiently. Already 1 h after preparation, all dispersions showed phase separation.
Based on these data, SDS in a concentration of 10% and a sonication time of 150 s had been chosen for the subsequent experiments.

### Figure 2: GPC elugram obtained from a BCA miniemulsion and evolution of elution time obtained from a PBCA dispersion during the course of four weeks.

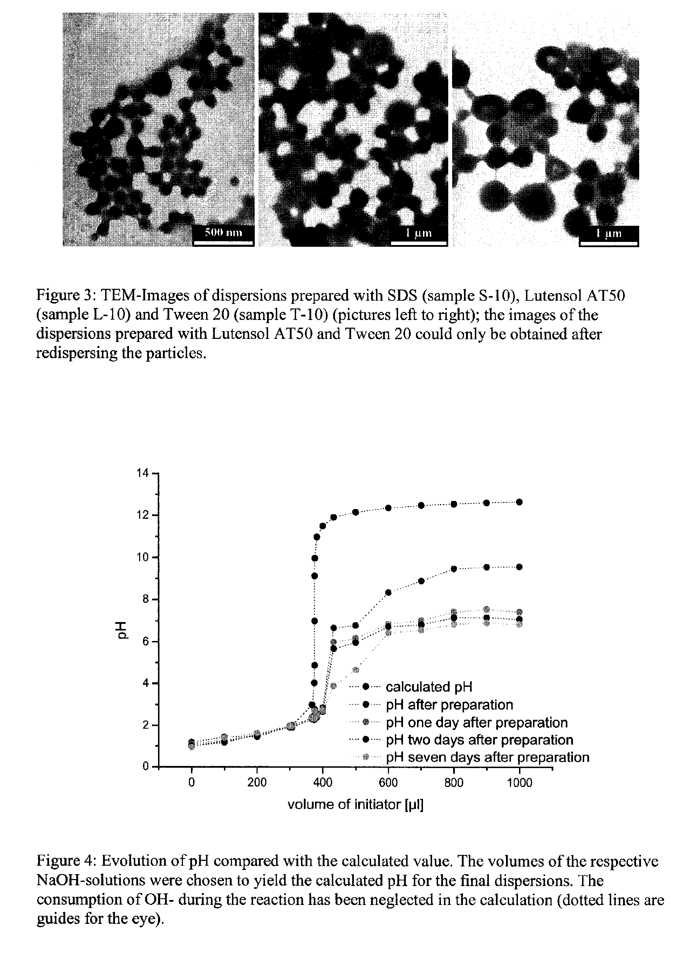

Figure 3 shows TEM micrographs from three selected dispersions. S-10 was prepared with 10 wt% SDS (150 s sonication, see marked sample in Table 1), L-10 with 10 wt% Lutensol AT50 and T-10 with 10 wt% Tween 20. The two latter samples could only be prepared after the sedimented dispersions had been shaken in order to redisperse the precipitate. The smaller size and greater uniformity of the particles prepared with SDS is clearly visible.

### Figure 3: TEM-Images of dispersions prepared with SDS (sample S-10), Lutensol AT50 (sample L-10) and Tween 20 (sample T-10) (pictures left to right); the images of the dispersions prepared with Lutensol AT50 and Tween 20 could only be obtained after redispersing the particles.

### 1.2 Influence of the amount of initiator NaOH on particle size and molecular weight

It is known from various BCA-emulsion polymerization experiments that the pH of the dispersion media affects the particle size and molecular weight {Behan, 2001 #29},{Lescure, 1992 #30}.

The influence of the pH on the characteristics of PBCA particles obtained in miniemulsion, are studied. The pH of the polymerization medium was adjusted by providing different amounts of NaOH solution to initiate the polymerization (see Table 2). It has been assumed that the neutralization reaction between the NaOH-solution and the HCl of the miniemulsion is fast compared to the initiation and the growth steps of the polymerization. In the case of miniemulsion, the polymerization pH could be increased to pH = 7. With the conventional emulsion technique, the pH of the polymerization could not be carried out at pH-values higher than 4-5, since at higher values polymerization is too fast and coagulum is formed{Behan, 2001 #29}.

**Table 2: Summary of dispersions initiated with NaOH.**

| **Volume of NaOH [µl]** | **Resulting pH (calculated)** | **Measured pH after seven days** | **Particle size (Z-Average)** | **Particle size (Z-Average) Measured after seven days** |
|---|---|---|---|---|
| 0 | 1.00 | 0.94 | 139 | 199 |
| 100 | 1.24 | 1.41 | 186 | 149 |
| 200 | 1.52 | 1.62 | 214 | 144 |
| 300 | 1.95 | 1.97 | 203 | 188 |
| 306.8 | 2.00 | 1.97 | 214 | 202 |
| 367.6 | 3.00 | 2.35 | 184 | 175 |
| 374.3 | 4.03 | 2.33 | 212 | 201 |
| 374.9 | 4.88 | 2.38 | 177 | 168 |
| 375 | 7.00 | 2.39 | 196 | 192 |
| 375.1 | 9.12 | 2.45 | 195 | 189 |
| 375.7 | 9.97 | 2.68 | 221 | 227 |
| 382.4 | 10.99 | 2.47 | 240 | 241 |
| 400 | 11.51 | 2.71 | 21.2 | 213 |
| 433.3 | 11.92 | 3.88 | 194 | 195 |
| 500 | 12.15 | 4.66 | 214 | 207 |
| 600 | 12.36 | 6.44 | 188 | 178 |
| 700 | 12.48 | 6.57 | 195 | 194 |
| 800 | 12.56 | 6.85 | 172 | 173 |
| 900 | 12.61 | 6.92 | 178 | 182 |
| 1000 | 12.66 | 6.85 | 180 | 183 |

Regarding the pH of the dispersion obtained in miniemulsion, it can be noticed that, especially considering calculated high pH-values, the values are constantly decreasing with time reaching a stable value after one day, which is below the expected value (Figure 4). This means, OH⁻ is consumed (about 10⁻² mol·l⁻¹) in an unexpectedly high amount during the reaction, which supports the assumption that OH⁻ is the initiating molecule, although it cannot be completely ruled out that Cl⁻ also acts as initiator.The particle size for all pH can be found in a narrow range between 150 and 220 nm, not considering the little volumes between 0 and 200 µl of initiator (because here the polymerization is too slow) which will be discussed separately, where no clear dependence can be observed. Since the values do not change significantly during the seven days, it can be assumed, that all of the dispersions are stable towards coagulation.

The values for the miniemulsion without the addition of a NaOH-solution (0 µl) show the evolution of the particle size in the "unperturbed" miniemulsion with an extremely slow polymerization. Directly after the preparation, when the first GPC measurement has been performed, it is reasonable to assume that the miniemulsion is still an emulsion and no polymer dispersion, since the pH of the minemulsion has an unaltered value of pH 1.0. With the dilution of the miniemulsion for the GPC measurement, polymerization will be initiated, so the actual droplet size may not be displayed correctly. Since the polymerization rate of the *n*-BCA in the miniemulsion system at the given pH (1.0) is unknown, it is impossible to determine the time of the "solidification" of the droplets. With the obvious growth of the droplets during the first day and the constant size between the first and the second day, the formation of size-stable polymer particles can be regarded as completed after this time. The heterophase initiation reaction leads to an increase in hydrophilicity of the oligomers by the attachment of OH- to the monomer, which in consequence allows the oligomers to diffuse through the continuous phase and can cause Ostwald ripening of the droplets explaining the growth of the droplets. After seven days, sedimentation is visible in the dispersion. This leads to a smaller detected particle size (and a narrower distribution) since the large particles are no longer included in the measurement. The same effect, but less pronounced, can be observed for the values for 100 and 200 µl of NaOH-solution.

Since the particles, prepared at higher initial pH do not show this decrease in particle size as distinctively it can be assumed that the conversion from droplet to particle had been completed after few minutes.

To summarize, for pH > 2, the particle size seems to be largely unaffected by the pH of the continuous phase during initiation and polymerization. Below this pH the time for the conversion from droplet to particle is lower than the time of droplet growth (Ostwald ripening).

### Figure 4: Evolution of pH compared with the calculated value. The volumes of the respective NaOH-solutions were chosen to yield the calculated pH for the final dispersions. The consumption of OH⁻ during the reaction has been neglected in the calculation (dotted lines are guides for the eye).

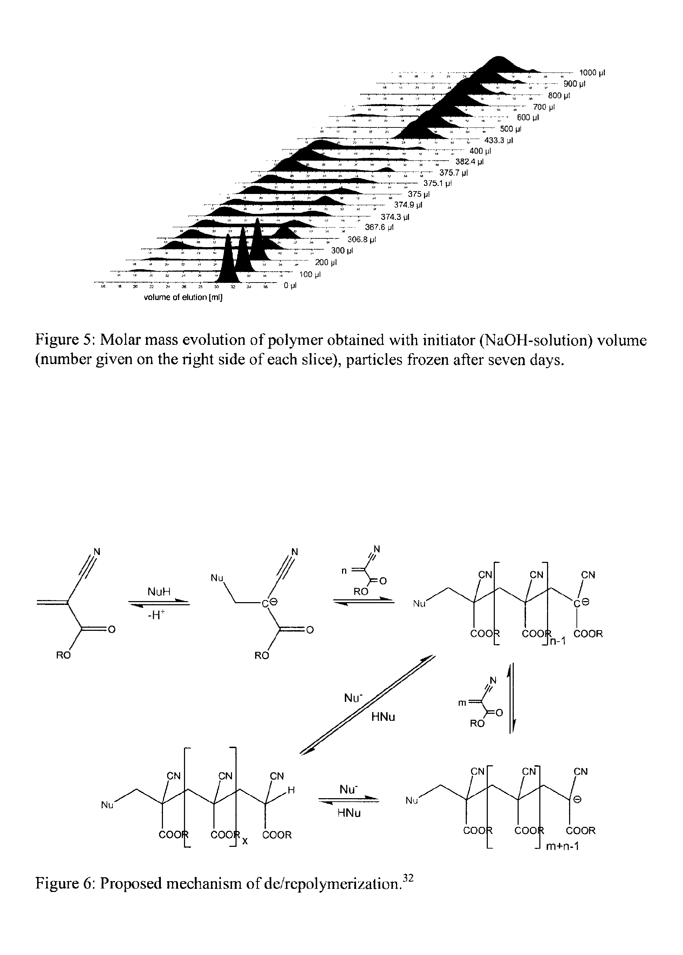

The GPC traces of the samples obtained with the addition of different NaOH amounts are presented in Figure 5. The molar masses of the polymers resulting from 0, 100 and 200 µl initiator solution show a narrow mass distribution (maximum at 4,000 g mol⁻¹) with very low amount of high molecular polymer. The polymers obtained with a volume of initiator between 300 and 400 µl (calculated pH 2.0 and 11.5) show a bimodal mass distribution with one maximum at about 4,000 gmol⁻¹ and one at 600,000 g mol⁻¹ (given to PS standard). A polymer fraction with masses between the extremes is only present in low amounts. The corresponding dispersions have a pH < 3 which is slightly lower than calculated. The rest of the samples correspond to dispersions with greater pH values, which are significantly lower than the calculated values (calculated 11.9 to 12.6, corresponding to the "plateau" in the pH diagram). The polymers formed under these conditions show monomodal mass distributions with a maximum at about 10,000 g mol⁻¹.

These results are similar to those of Behan et al. {Behan, 2001 #29} who obtained particles prepared by the conventional emulsion polymerization using dextran as steric stabilizer. As long as the polymerization is carried out in an initially acidic medium (pH 2-3), polymer with a relatively low molecular weight is obtained, whereas long chains additionally appear as soon as the polymerization medium has an initially higher pH (pH 5). Although the values obtained by Behan et al. {Behan, 2001 #29}, {Behan, 2000 #14} are lower than the values presented in this paper the general tendencies are the same.

### Figure 5: Molar mass evolution of polymer obtained with initiator (NaOH-solution) volume (number given on the right side of each slice), particles frozen after seven days.

It has to be emphasized that the freezing of the dispersions has been performed seven days after preparation. This is of great importance, since it could be shown for one pH (initially 7), that the initial mass distribution changes during the days after preparation (see Figure 2). Therefore a large amount of dispersion has been prepared. The particle size was 125 nm.

The freeze dried miniemulsion shows a peak in the GPC elugram at approximately 33.3 ml, corresponding to a mass of about 1,400 g-mol⁻¹ (calibrated to PS standard). This means that either in miniemulsion (possible initiation by Cl⁻) or in the frozen miniemulsion polymerization has been initiated, since no NaOH solution has been added. Immediately after adding the miniemulsion to the NaOH solution polymer with an *M*_{w} maximum at about 10,000 g-mol⁻¹ (elution volume approximately 29 ml) is formed. No change in molar mass distribution is visible during the course of 48 h. After this time high and low molecular weight polymer is beginning to be formed. The oligomers formed are signs of an ongoing depolymerization process. These oligomers disappear, whereas the amount of the long chain polymer is increasing until the final distribution is reached after approximately one week. The maximum of the low molecular weight fraction shifts to a value of 7,500 g-mol⁻¹ (elution volume approximately 30 ml).

These observations are in accordance and can be explained with the pH dependent de/repolymerization/reinitiation mechanism proposed by Ryan as shown in Figure 6 {Ryan, 1996 #37}.

### Figure 6: Proposed mechanism of de/repolymerization. {Ryan, 1996 #37}

### Amine initiators

The anionic polymerization of ACAs is initiated by nucleophiles. Even "weak" nucleophiles like acetate ions possess the ability of initiating the polymerization of ACAs {Pepper, 1992 #45}, {Johnston, 1981 #48; Johnston, 1981 #49; Johnston, 1981 #50}. As mentioned briefly above, the growing polymer is functionalized by the initiator molecule. If a nanoparticle is formed out of such a functionalized polymer and it can be ensured that the functional group (due to its hydrophilicity) will be at the particles' surface, this approach presents a convenient way to prepare PACA (nano)particles with functionalized surfaces.

The surface tailoring of nanoparticles greatly enhances their potential for biomedical applications. The presence of functional groups on the surface is required for further chemical modification with bioactive ligands like proteins or nucleotides. Besides the potential for further chemical reaction the introduction of charged groups like amino or carboxylic acid groups, influences the particles' surface charge. This as a consequence can affect the particles' stability in dispersion {Chern, 2001 #51} and the uptake behaviour in cells {Lorenz, 2006 #52}, {Holzapfel, 2005 #53}.

With the application of polar, hydrophilic amines, the resulting oligomers and the polymer will have a surfactant like amphiphilic structure, with a hydrophilic head, originating from the initiating amine and a hydrophobic tail - the (growing) polymer. Due to this structure, it is very likely that the hydrophilic functionalized end of the polymer can be found on the aqueous side of the interface between monomer and water.

Bifunctional amines allow the further introduction of functional groups to the particles' surface. With the scope of biomedical application and the potential conjugation of proteins to the particles amino acids are the appropriate candidates as initiators {Kulkarni, 1971 #43; Leonard, 1966 #44}, since they incorporate a "strong" nucleophile (-NH₂) which will be the initiating part of the molecule and the "weakly" nucleophilic part (-COOH), which at least in its protonated form is not likely to initiate polymerization and is therefore available for subsequent chemical reactions.

As shown above the amount of initiator OH- is crucial for the molar mass of the polymer whereas the particle size is in a close range over all applied pH values. The influence of the various amounts of amine initiator on these parameters will be discussed in this part.

### Ammonia and Tris-Base

As model amines ammonia and tris-base (tris(hydroxyethyl)aminoethane) have been chosen. Solutions with a concentration of 0.1 mol 1⁻¹ each have been prepared without adjusting their pH-values (pH > 9). This means, that besides the amine in solution there is also OH- present. Thus there will be competition between the amine and the hydroxyl ions for the initiation of the polymerization. The particle sizes and PDIs are summarized in Table 3. The same volumes of initiator solutions as in the experiments with OH⁻ have been chosen. Some of the samples prepared with ammonia (600 µl -1000 µl) show a slight yellow coloring after preparation. This has been observed by Leonard {Leonard, 1966 #44} and been interpreted as reaction products after hydrolysis of the butyl ester group.

In contrast to the sizes of the particles prepared with NaOH-solution, which are more or less unaffected by the concentration of the initiator, a clear dependence on the concentration of the amine initiator can be observed. The sizes of the polymer particles obtained with both amine solutions follow the same pattern; the particles prepared with ammonia are smaller than the particles prepared with tris-base solution. Each of the 100 µl samples exhibit particle sizes significantly larger than the rest of the samples. After a steep decrease the values reach a minimum at about 400 µl initiator solution and increase again.

The comparably large particles obtained with initiator volumes of 100 µl and for tris-base also 200 µl can be explained, as for OH⁻, with the longer solidification time of the particles.

The diameters of the particles prepared with amines are significantly smaller than those of the particles prepared with NaOH-solution. Instead of values around 200 nm the particles range from 60 to 100 nm, respectively from 100 to 140 nm. This might be a consequence of the additional stabilization due to the surfactant like structure of the polymer.

**Table 3: Particle sizes of dispersions initiated with ammonia and tris-base solution (measured after polymerization).**

| Volume of initiator solution solution [µl] | ammonia solution 0.1 mol l⁻¹ | | Tris base 0.1 mol l⁻¹ | |
|---|---|---|---|---|
| | Particle size (z-average) [nm] | PDI | Particle size (z-average) [nm] | PDI |
| 100 | 233 | 0,236 | 244 | 0,168 |
| 200 | 86 | 0,234 | 164 | 0,196 |
| 300 | 64 | 0,268 | 104 | 0,261 |
| 306.8 | 47 | 0,242 | 105 | 0,239 |
| 367.6 | 69 | 0,212 | 100 | 0,233 |
| 374.3 | 67 | 0,198 | 98 | 0,223 |
| 374.9 | 68 | 0,213 | 97 | 0,213 |
| 375 | 65 | 0,261 | 96 | 0,205 |
| 375.1 | 67 | 0,245 | 95 | 0,223 |
| 375.7 | 70 | 0,263 | 94 | 0,218 |
| 382.4 | 72 | 0,265 | 95 | 0,228 |
| 400 | 77 | 0,243 | 99 | 0,228 |
| 433.3 | 70 | 0,252 | 98 | 0,213 |
| 500 | 84 | 0,237 | 115 | 0,184 |
| 600 | 97 | 0,192 | 125 | 0,152 |
| 700 | 96 | 0,203 | 132 | 0,132 |
| 800 | 95 | 0,184 | 137 | 0,109 |
| 900 | 98 | 0,191 | 141 | 0,074 |
| 1000 | 111 | 0,152 | 145 | 0,101 |

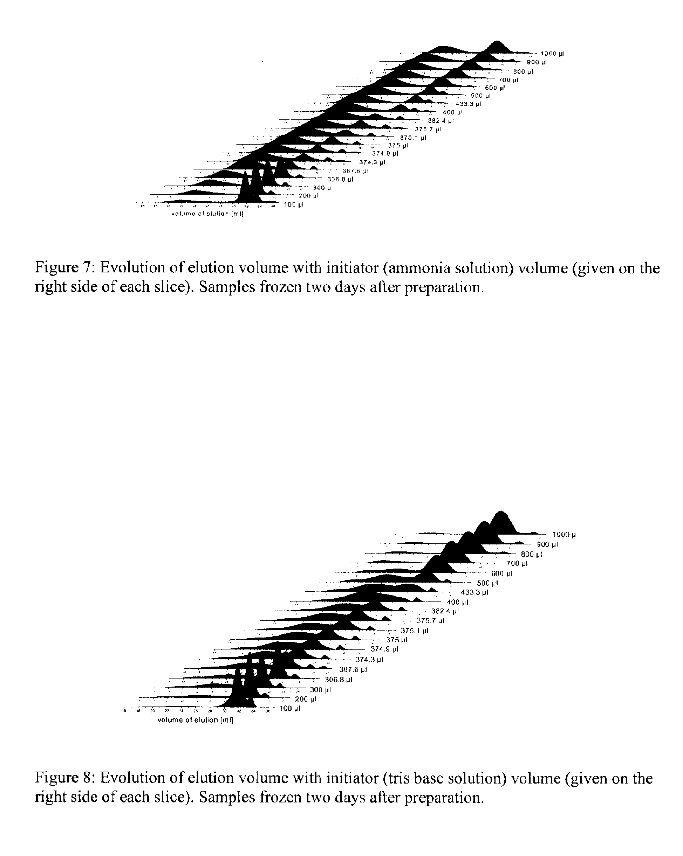

Molar mass distributions for the ammonia-initiated polymers (see Figure 7) differ from the values obtained from the OH-initiated polymers. The main difference is the appearance of high molar mass polymer in all samples. The amount is increasing from 100 µl to 306.8 µl initiator solution. The distribution is broad and shows only minor changes throughout the samples. The low molar weight fraction (maximum at 32 ml elution volume, *M*_{w}~3,500 g mol⁻¹) is decreasing constantly form 100 µl to 382.4µl with a shift of the maximum of the elution volume to 29 ml (*M*_{w}~10,000 g mol⁻¹). With the application of more initiator solution the amount of low molecular weight fraction increases again and shows its maximum at approximately 31 ml (*M*_{w}~5,000 g mol⁻¹).

The molar mass distributions of the polymers initiated with tris-base solution (see Figure 8) resemble the pattern of the OH-initiated polymer samples as well as the ammonia-initiated ones. Large amounts of long chain polymers can only be found at intermediate amounts of initiators, showing a broad distribution with values lower than those obtained in the other sets. The maxima of the low molar mass fraction can be found at approximately 31 ml (*M_{w}*~5,000 g mol⁻¹). The relative amount of this fraction drops constantly from 100 µl to 443.3 µl then increases again to reach a maximum at 1000 µl of initiator solution.

### Figure 7: Evolution of elution volume with initiator (ammonia solution) volume (given on the right side of each slice). Samples frozen two days after preparation.

### Figure 8: Evolution of elution volume with initiator (tris base solution) volume (given on the right side of each slice). Samples frozen two days after preparation.

### Amino Acids

For a functionalization of the polymer with amino acids, it has to be assured, that the amino acid is the sole, or at least the main initiating molecule. Thus the pH of the amino acid solution has to be low in order to minimize the inititation of the polymerization by the hydroxy ions. This, on the other hand protonates the amino groups to a certain extent, determined by its pK_{B}, lowering the amount of "active" initiator, namely an amino acid molecule with deprotonated amino group.

Stable dispersions could be created using phenylalanine in acidic solution, glycine and 6-aminohexanoic acid in acidic as well as in basic solutions. Lysine, cysteine, arginine, glutamic and aspartic acid solutions with pH values lower than 7 led always to coagulation and precipitation of the miniemulsion. In basic solutions no coagulum was formed but the formation of clear yellow or orange colored solutions could be observed. The dissolution and the coloring is a clear sign for hydrolysis of the butylester group and the formation of water soluble polycyanoacrylic acid. The coloring is according to Leonard { Leonard, 1966 #44} also a sign for degradation of the PACA.

**Table 5: Set of dispersion initiated with 0.5 mol l⁻¹ 6Ahex solution, 0.1, 0.5, and 2.0 mol l⁻¹ glycine solution (first value: z-averaged diameter, second value: PDI)**

| Volume of initiator [µl] | 6AHex 0.5 mol l⁻¹ | | | Gly 0.1 mol l⁻¹ | | | | Gly 0.5 mol l⁻¹ | | | Gly 2.0 mol l⁻¹ | | |
|---|---|---|---|---|---|---|---|---|---|---|---|---|---|
| | pH 4.4 | pH 5.4 | pH 10.0 | pH 2.4 | pH 3.4 | pH 4.4 | pH 5.4 | pH 3.4 | pH 4.4 | pH 5.4 | pH 3.4 | pH 4.4 | pH 5.4 |
| 100 | Coagula tion | 129 | 83 | 360 | 336 | 302 | 297 | 184 | 204 | 232 | 165 | 121 | 108 |
| | | 0.089 | 0.220 | 0.134 | 0.163 | 0.127 | 0.109 | 0.056 | 0.075 | 0.108 | 0.016 | 0.042 | 0.050 |
| 200 | 238 | 100 | 70 | 270 | 223 | 202 | 198 | 146 | 170 | 156 | 144 | 117 | 107 |
| | 0.107 | 0.141 | 0.200 | 0.151 | 0.084 | 0.035 | 0.043 | 0.019 | 0.002 | 0.082 | 0.018 | 0.049 | 0.018 |
| 300 | 192 | 93 | | 223 | 189 | 164 | 162 | 137 | 158 | 127 | 139 | 115 | 103 |
| | 0.010 | 0.161 | | 0.062 | 0.052 | 0.055 | 0.062 | 0.045 | 0.010 | 0.047 | 0.030 | 0.040 | 0.041 |
| 400 | 195 | 89 | 72 | 204 | 159 | 142 | 146 | 136 | 149 | 124 | 134 | 111 | 97 |
| | 0.141 | 0.167 | 0.199 | 0.094 | 0.037 | 0.069 | 0.057 | 0.035 | 0.013 | 0.026 | 0.020 | 0.057 | 0.059 |
| 500 | 185 | 87 | | 187 | 139 | 132 | 141 | 137 | 144 | 122 | 129 | 107 | 92 |
| | 0.042 | 0.190 | | 0.043 | 0.074 | 0.074 | 0.032 | 0.085 | 0.040 | 0.022 | 0.070 | 0.071 | 0.089 |
| 600 | 178 | 86 | 76 | 176 | 126 | 130 | 139 | 129 | 145 | 120 | 126 | 106 | 88 |
| | 0.065 | 0.182 | 0.195 | 0.045 | 0.063 | 0.052 | 0.006 | 0.045 | 0.035 | 0.003 | 0.029 | 0.062 | 0.093 |
| 700 | 187 | 85 | | 166 | 125 | 128 | 138 | 128 | 142 | 118 | 124 | 105 | 87 |
| | 0.105 | 0.174 | | 0.052 | 0.088 | 0.086 | 0.059 | 0.048 | 0.010 | 0.013 | 0.063 | 0.043 | 0.110 |
| 800 | 182 | 86 | 81 | 161 | 118 | 130 | 139 | 127 | 137 | 116 | 121 | 104 | 85 |
| | 0.209 | 0.183 | 0.198 | 0.054 | 0.058 | 0.080 | 0.065 | 0.030 | 0.035 | 0.048 | 0.087 | 0.044 | 0.121 |
| 900 | 202 | 84 | | 154 | 122 | 135 | 154 | 127 | 135 | 118 | 120 | 104 | 83 |
| | 0.197 | 0.180 | | 0.064 | 0.045 | 0.014 | 0.080 | 0.016 | 0.016 | 0.040 | 0.058 | 0.066 | 0.110 |
| 1000 | 173 | 85 | 83 | 152 | 121 | 126 | 137 | 125 | 132 | 119 | 117 | 102 | 85 |
| | 0.044 | 0.172 | 0.174 | 0.029 | 0.044 | 0.061 | 0.064 | 0.025 | 0.046 | 0.022 | 0.082 | 0.060 | 0.092 |

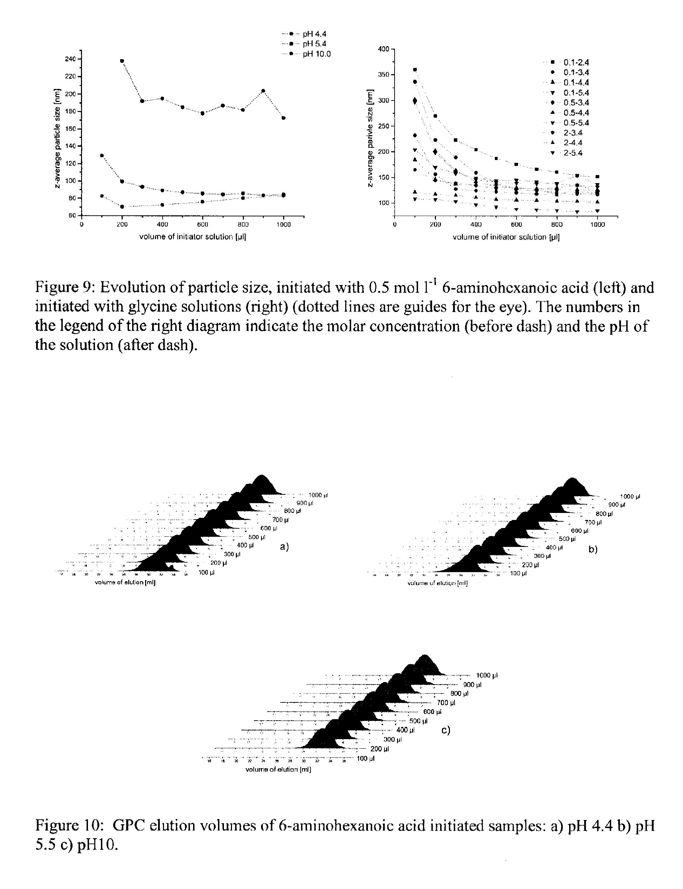

The data available in Table 5and visualized in Figure 9 shows, that the particle sizes cover the range from more than 350 nm to values as low as 70 nm. Nearly all of the dispersions show an extremely narrow distribution, expressed by the PDI smaller than 0.1 or even below.

In contrast to the experiments with NaOH-solution as initiator and in accordance to the results of the particles prepared with ammonia and tris-base a clear dependence of the particle size on the amount of "active" initiator can be observed. Higher concentration, higher pH and greater amount of the initiator solution lead to smaller particles in almost all series.

Regarding the sizes of the particles prepared with the 6-aminohexanoic acid solutions a dependence on the pH of the initiator and to some extent on the amount of initiator is visible (see Figure 9 left). The particle size increases with increasing pH of the initiator solution, with a large effect between pH 4.4 and 5.4 and a smaller between pH 5.4 to 10.0. The sizes for particles prepared with the basic (pH 10) 6-aminohexanoic acid solution show a similar evolution as the values for the particles prepared with tris-base and ammonia solution. After a decrease in particle size from 100 µl to 200 µl initiator solution the curve shows a slight increase with the highest value for 1000 µl initiator solution. The particles prepared with the other 6-aminohexanoic acid solutions also show the decrease in size from the initial to the following volume of initiator solution. The subsequent values remain almost constant.

The values for the particles prepared with the glycine solutions follow in most cases the above mentioned pattern (see Figure 9 right). The particles prepared with the 2 mol l⁻¹ glycine solution appear as the smallest whereas the particles prepared with 0.1 mol l⁻¹ solution show the largest size values. Within one concentration the particle size decreases with the exception of 0.5 mol l⁻¹ solution from lowest to highest applied pH. The same tendency can be observed from 100 µl to 1000 µl initiator solution. The slope of the curves becomes less steep with increasing pH and concentration of glycine solution.

### Figure 9: Evolution of particle size, initiated with 0.5 mol l⁻¹6-aminohexanoic acid (left) and initiated with glycine solutions (right) (dotted lines are guides for the eye). The numbers in the legend of the right diagram indicate the molar concentration (before dash) and the pH of the solution (after dash).

The oligomers resulting from the reaction of the amino acid and the monomer are expected to be water soluble because of the high hydrophilicity of the amino acid. Therefore the polymerization is not restricted to one droplet and the droplets undergo Ostwald ripening as long as the solidification has not started or the hydrophobicity of the PBCA chain dominates and the molecules are no longer soluble in the aqueous phase. With a low amount of initiated polymer chains, the solidification will take a longer time than with more growing chains present. During this time the particles can increase their size. Despite this effect a narrow particle size distribution can be observed at all samples. The possible occurrence of micelles formed of surfactant like oligomer might even complicate the process of particle formation.

If the polymerization time is excessively high, the droplets can exceed a critical size, which leads to coagulation and after polymerization precipitation of the particles. Thus, no stable dispersions could be obtained using 100 µl of 6-aminohexanoic acid solution at pH 4.4 and glycine solutions with concentrations of 0.5 and 2.0 mol 1⁻¹ at pH 2.4.

Despite this variation in particle size all polymer samples show nearly the same molecular mass (maxima at approximately 4,000 g mol⁻¹ calibrated against PS) and mass distribution, which is narrow and monomodal. With the molar mass of 4,000 g mol⁻¹ the degree of polymerization is about *P* ~ 26. This means that the ratio between monomer and initiator is near 1:25 (see below ¹H NMR). Regarding the uniform elugrams this must be true for all samples. In contrast to the particles prepared with basic initiator solution (NaOH, NH₃ and tris-base) no significant variation in mass distribution between the samples can be observed with the variation of the initiator volume. This implicates an independence of pH, concentration and volume of initiator at least in the examined ranges. There is even only minor deviation between the two amino acids used for preparation.

### Figure 10: GPC elution volumes of 6-aminohexanoic acid initiated samples: a) pH 4.4 b) pH 5.5 c) pH10.

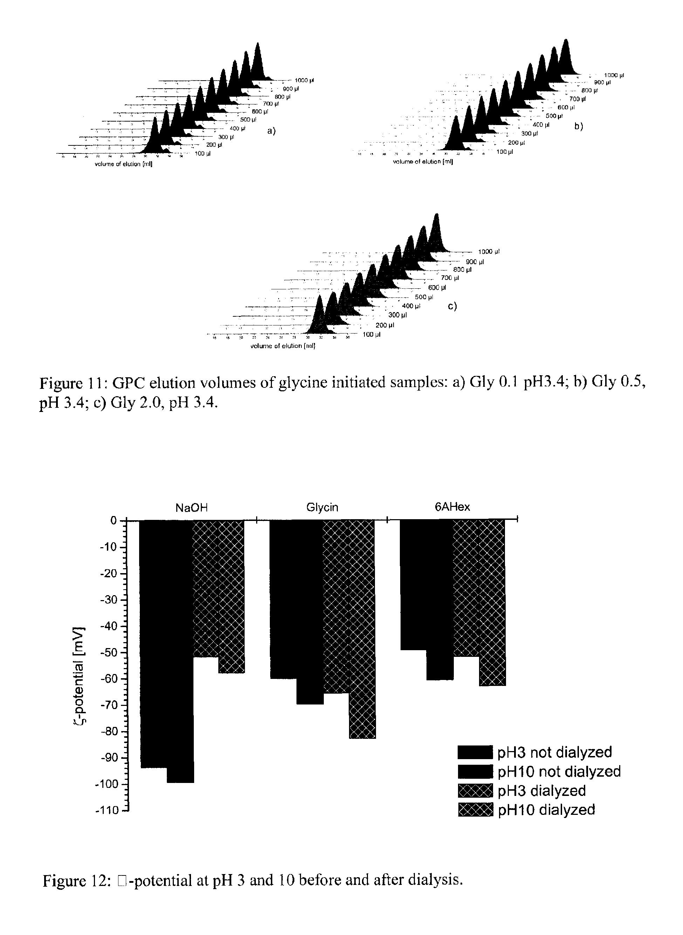

### Figure 11: GPC elution volumes of glycine initiated samples: a) Gly 0.1 pH3.4; b) Gly 0.5, pH 3.4; c) Gly 2.0, pH 3.4.

As shown in Figure 12, the pH-dependence of the ζ-potential is clearly visible for the samples prepared with amino acids. The ζ-potential of the particles prepared with amino acids is ~ 10 mV higher in acidic medium (pH 3) than in basic medium (pH 10). The particles prepared with NaOH-solution also show a ζ-potential with a slight pH-dependence. This is not surprising, since in basic medium hydrolysis of the butyl ester groups is likely to occur. Compared with the particles prepared with amino acid solutions this effect is not as pronounced.

The increase of ~50 mV of the ζ-potential is due to the removal of SDS (by dialysis) which is adsorbed on the particles. More thorough dialysis leads to further removal of the adsorbed SDS which destabilizes the dispersion and leads to the formation of precipitate. This effect cannot be observed on the samples prepared with amino acid solutions. In contrast a slight decrease of the ζ-potential occurs after dialysis. Since the dispersion is diluted during dialysis the pH of the dispersion increases and resulting hydrolysis of the ester groups can create additional negative charge on the particles.

As soon as there are carboxy-groups present on the particle surface a pH-dependence of the ζ-potential should be observable. In basic medium, the acid group is deprotonated leaving a negative charge on the particle, in acidic medium this negative charge is no longer present, since the acid group is protonated. Therefore ζ-potential measurements have been performed after adjusting the pH of the diluted dispersion to values of 3 and 10, respectively. Still, there is the possibility that the amino acids are only adsorbed to the particles' surface. To exclude this possibility the dispersions have been dialyzed, in order to remove adsorbed molecules and to some extent SDS.

### Figure 12: ζ-potential at pH 3 and 10 before and after dialysis.

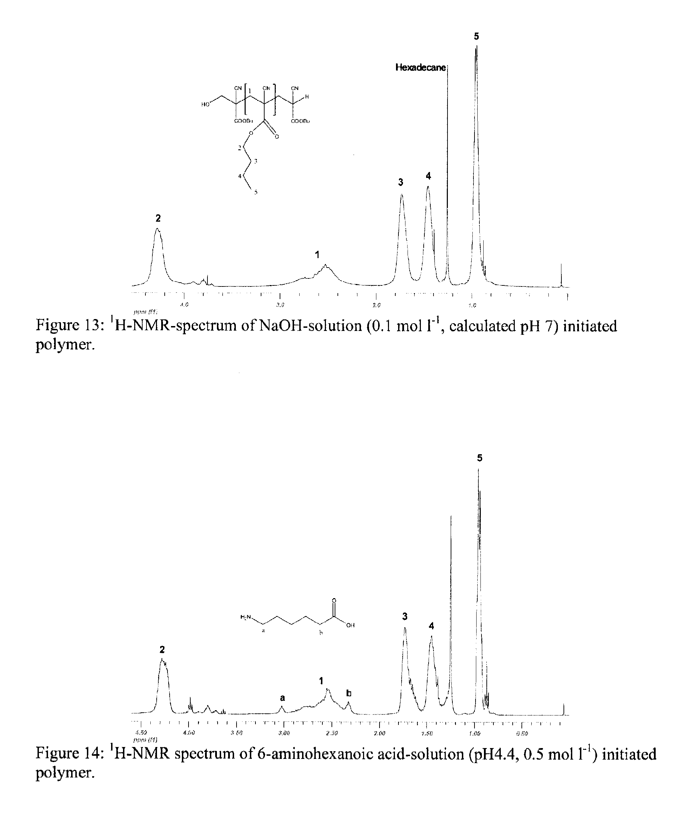

Figures 13 and 14 show ¹H-NMR spectra of the polymer obtained from a dispersion initiated with NaOH-solution (0.1 mol l⁻¹) and with 6-aminohexanoic acid-solution (pH 4.4, 0.5 mol l⁻¹). The peaks of the spectrum in Figure 13 can be identified and assigned to the polymer and the hexadecane. The spectrum presented in Figure 14 shows two additional peaks which might originate from protons of the 6-aminohexanoic acid. Since 6-aminohexanoic acid is poorly soluble in CDCl₃, the acid must be covalently bound to the polymer in order to be detected by NMR. The ratio of the integrals of peaks a:3, representing the ratio amino acid to monomer is app. 1:22, which is in quite good accordance to the ratio of 1:25 obtained from the GPC values.

### Figure 13: ¹H-NMR-spectrum of NaOH-solution (0.1 mol l⁻¹, calculated pH 7) initiated polymer.

### Figure 14: ¹H-NMR spectrum of 6-aminohexanoic acid-solution (pH4.4, 0.5 mol l⁻¹) initiated polymer.

A direct proof for the attachment of the amino acids to the polymer gave the application of phenylalanine as initiator. Since pBCA shows no UV activity, it can only be detected by the RI-detector of the GPC setup, but not by the UV-detector. The introduction of the UV-active initiator phenylalanine labels the polymer chain, so that it can be observed by the UV-detector. Since the signals of both detectors are nearly congruent for the phenylalanine initiated polymer and it can be assumed, that the UV signal originates from the phenylalanine, the amino acid has to be covalently bound to the polymer.

Unfortunately, it was not possible to observe the particles directly via electron microscopy. The particles tend to form a film during drying, which makes it impossible to prepare TEM-samples. Even during freeze drying the film formation process is occurring. Instead of obtaining a fine powder, as it is possible with the dispersions prepared with NaOH-solution, an off-white gumlike mass is the result of the attempted freeze-drying of the dispersion prepared with amino acid solutions.

Low molecular weight, strong adsorption of water by the functionalized polymer or residual monomer {Behan, 2001 #29} might be the reasons for this effect.

### Conclusion

It could be shown that the miniemulsion approach provides a very powerful and convenient tool to produce pBCA-nanoparticles with functionalized surfaces. The application of the anionic surfactant SDS allows the preparation of long-term stable dispersions with solid contents of 10 % or more and leads to "true" pBCA particles without covalently bound dextran or other steric stabilizer on the surface. The two-step process extends the pH of polymerization and therefore allows obtaining pBCA of comparably high molecular weight. The particle sizes obtained are largely unaffected by the pH and can be found between 150 and 200 nm.

Initiation with amine solutions provides an easy way of introducing functional groups to the particles' surface. The application of amino acid solutions as initiators gives rise to the possibility to functionalize the particles and tune the particle size in the range between 80 and 350 nm. The presence of amino acids has been confirmed by ζ-potential measurements and by NMR.Preliminary experiments show that the encapsulation of fluorescent dye, vitamins as well as inorganic nanoparticles is feasible with this technique.

Literature:
1. Arias, J. L.; Gallardo, V.; Gómez-Lopera, S. A.; Plaza, R. C.; Delgado, A. V. Journal of Controlled Release 2001, 77, 309-321.
2. Reddy, L. H.; Murthy, R. R. Acta Pharm. 2004, 51, 103-118.
3. Kattan, J.; Droz, J.-P.; Couvreur, P.; Marino, J.-P.; Boutan-Laroze, A.; Rougier, P.; Brault, P.; Vranckx, H.; Grognet, J.-M.; Morge, X.; Sancho-Garnier, H. Investigational New Drugs 1992, 10, 191-199.
4. Steiniger, S. C. J.; Kreuter, J.; Khalansky, A. S.; Skidan, I. N.; Bobruskin, A. I.; Smirnova, Z. S.; Severin, S. E.; Uhl, R.; Kock, M.; Geiger, K. D.; Gelperina, S. E. Int. J. Cancer 2004, 109, 759-767.
5. Gulyaev, A. E.; Gelperina, S. E.; Skidan, I. N.; Antropov, A. S.; Kivman, G. Y.; Kreuter, J. Pharmaceutical Research 1999, 1564-1569.
6. Alyautdin, R.; Gothier, D.; Petrov, V.; Kharkevich, D.; Kreuter, J. European Journal of Pharmaceutics and Biopharmaceutics 1995, 41, (1), 44-48.
7. Olivier, J.-C.; Fenart, L.; Chauvet, R.; Pariat, C.; Ceccelli, R.; Couet, W. Pharmaceutical Research 1999, 16, (12), 1836-1842.
8. Behan, N.; Birkinshaw, C. Macromolecular Rapid Communications 2001, 22, 41-43.
9. Sullivan, C. O.; Birkinshaw, C. Biomaterials 2004, 25, 4375-4382.
10. Couvreur, P. CRC Critical Revievs in Therapeutic Drug Carrier Systems 1988, 5, (1), 1-17.
11. Couvreur, P.; Kante, B.; Roland, M.; Speiser, P. Journal of Pharmaceutical Sciences 1979, 68, (12), 1521-1524.
12. Limouzin, C.; Caviggia, A.; Ganachaud, F.; Hémery, P. Macromolecules 2003, 36, 667-674.
13. Behan, N.; Birkinshaw, C.; Clarke, N. Biomaterials 2001, 22, 1335-1344.
14. Lescure, F.; Zimmer, C.; Roy, D.; Couvreur, P. Journal of Colloid and Interface Science 1992, 154, (1), 77-86.
15. El-Egakey, M. A.; Bentele, V.; Kreuter, J. International Journal of Pharmaceutics 1983, 13, 349-352.
16. Douglas, S. J.; Illum, L.; Davis, S. S.; Kreuter, J. Journal of Colloid and Interface Science 1984, 101, (1), 149-158.
17. Douglas, S. J.; Illum, L.; Davis, S. S. Journal of Colloid and Interface Science 1985, 103, (1), 154-163.
18. Vasnick, L.; Couvreur, P.; Christiaens-Leyh, D.; Roland, M. Pharmaceutical Research 1985,36-41.
19. Seijo, B.; Fattal, E.; Roblot-Treupel, L.; Couvreur, P. International Journal of Pharmaceutics 1990, 62, 1-7.
20. Labib, A.; Lenaerts, V.; Chouinard, F.; Leroux, J.-C.; Oullet, R.; van Lier, J. E. Pharmaceutical Research 1991, 8, (8), 1027-1031.
21. Chauvierre, C.; Vauthier, C.; Labarre, D.; H., H. Colloid Polym Sci. 2004, 282, 1016-1025.
22. Peracchia, M. T.; Vauthier, C.; Passirani, C.; Couvreur, P.; Labarre, D. Life Sciences 1997, 61, (7), 749-761.
23. Lathia, J. D.; El-Sherif, D.; Dhoot, N. O.; Wheatley, M. A. Pharmaceutical Engineering 2004, 24, (1), 1-8.
24. Nakajima, N.; Ikada, Y. Bioconjugate Chem. 1995, 6, 123-130.
25. Rasmussen, S. R.; Larsen, M. R.; Rasmussen, S. E. Analytical Biochemistry 1991, 198, 138-142.
26. Pepper, D. C. Journal of Polymer Science: Polymer Symposium 1978, 62, 65-77.
27. Kulkarni, R. K.; Bartak, D. E.; Leonard, F. Journal of Polymer Science: Part A-1 1971, 9, 2977-2981.
28. Leonard, F.; Kulkarni, R. K.; Brandes, G.; Nelson, J.; Cameron, J. J. Journal of Applied Polymer Science 1966, 10, (259-272).
29. Pepper, D. C.; Ryan, B. Makromol. Chem. 1983, 184, 383-394.
30. Costa, G.; Cronin, J. P.; Pepper, D. C.; Loonan, C. Eur. Polym. J. 1983, 19, (10/11), 939-945.
31. Behan, N.; Birkinshaw, C. Macromolecular Rapid Communications 2000, 21, 884-886.
32. Ryan, B.; McCann, G. Macromolecular Rapid Communications 1996, 17, 217-227.
33. Pepper, D. C. Makromol. Chem., Macromol Symp. 1992, 60, 267-277.
34. Johnston, D. S.; Pepper, D. C. Makromol. Chem. 1981, 182, 393-406.
35. Johnston, D. S.; Pepper, D. C. Makromol. Chem. 1981, 182,421-435.
36. Johnston, D. S.; Pepper, D. C. Makromol. Chem. 1981, 182, 407-420.
37. Chern, C.-S.; Sheu, J.-C. Polymer 2001, 42, 2349 - 1357.
38. Lorenz, M. R.; Holzapfel, V.; Musyanovych, A.; Nothelfer, K.; Walther, P.; Frank, H.; Landfester, K.; Schrezenmeier, H.; Mailänder, V. Biomaterials 2006, 27, 2820-2828.
39. Holzapfel, V.; Musyanovych, A.; Landfester, K.; Lorenz, M. R.; Mailänder, V. Macromol. Chem. Phys 2005, 206, 2440-2449.

## Claims

1. A method of producing polyalkylcyanoacrylate (PACA) nanoparticles comprising the steps of:
a) preparing an O/W miniemulsion, comprising O and W type liquid phases, a stabilizer, and polymerizable ACA monomers,
b) polymerizing said monomers by anionic polymerization, and isolating the produced nanoparticles,
**characterized in that**
the polymerization in step b) is initiated by one or more primary or secondary amines.

2. The method of claim 1, wherein the primary or secondary amine is selected from the group consisting of ammonia, tris-base, or from amino acids, preferably phenylalanine, glycine or 6-aminohexanoic acid.

3. The method of claim 1 or 2, wherein one or more pharmaceutical agents are contained in the W and/or in the O phase, preferably selected from a therapeutic agent and a diagnostic agent.

4. The method of claim 3, wherein the therapeutic agent is selected from substances which are incapable or not sufficiently capable of crossing physiological barriers without a delivery vehicle or carrier, wherein the physiological barrier preferably is selected from the group consisting of blood-brain barrier (bbb), blood-air barrier, blood-cerebrospinal fluid barrier and buccal mucosa.

5. The method of one or more of the preceding claims, wherein the O phase comprises a lipophilic solvent, preferably n-hexane, hexadecane, liquid paraffin, vitamine E, miglyol or fatty acid esters of triglycerides

6. The method of one or more of the preceding claims, wherein the polymeric material obtained form the monomers is biodegradable and comprises solid or film forming polymers, selected from the group consisting of polyalkylcyanoacrylates, more preferably polybutylcyanoacrylates and derivatives, copolymers and mixtures thereof.

7. The method of one or more of the preceding claims, wherein the stabilizer comprises one or more of the following substances:
fatty acid esters of glycerols, sorbitol and other mono- or multifunctional alcohols, preferably benzyl alcohol, glycerol monostearate, sorbitan monolaurate, or sorbitan monoleate; phospholipids, phosphoric acid esters, polysaccharide, benzyl benzoate, polyethylene glycol (PEG 200, 300, 400, 500, 600), polyethylene glycol hydroxystearate, preferably Solutol HS 15; poloxamines, preferably poloxamine 904, 908 or 1508; polyoxyethylene ethers and polyoxyethylene esters; ethoxylated triglycerides; ethoxylated phenols and ethoxylated diphenols; surfactants of the Genapol TM and Bauki series; polyoxyl castor oils, preferably Cremophor ELP; lecithin, metal salts of fatty acids, metal salts of fatty alcohol sulfates; and metal salts of sulfosuccinates; preferably polysorbates, more preferably polysorbate 20, 60 and most preferably polysorbate 80; preferably poloxamers, more preferably poloxamer 188, 338 or 407; preferably polyoxyethylene glycols, more preferably Lutensol 50 or 80; anionic surfactants, for example sodium dodecyl sulfate; and mixtures of two or more of said substances.

8. Nanoparticles obtainable by the method of one or more of claims 1-7.

9. A pharmaceutical composition, comprising the nanoparticles of claim 8 and a pharmaceutically acceptable carrier and/or diluent.

10. Use of the nanoparticles of claim 8 or the pharmaceutical composition of claim 9 for the manufacture of a medicament for the treatment of diseases and conditions, requiring a pharmaceutical agent to cross one or more physiological barriers, preferably the blood-brain barrier.
